# FASCICULE DE BREVET EUROPEEN

(11) **EP 1 999 145 B1**
(45) Date de publication et mention de la délivrance du brevet: **11.12.2013**
(21) Numéro de dépôt: 07731833.5
(22) Date de dépôt: 27.03.2007
(51) Int. Cl.: C07K 14/47, A61M 1/34

(54) **UTILISATION DE FILTRES DE DÉLEUCOCYTATION POUR LA PURIFICATION DE DÉFENSINES**
VERWENDUNG VON DELEUKOZYTATIONSFILTERN ZUR AUFREINIGUNG VON DEFENSIN
USE OF DELEUCOCYTATION FILTERS FOR DEFENSIN PURIFICATION

(30) Priorité: 28.03.2006 FR 0651067
(43) Date de publication de la demande: 10.12.2008
(73) Titulaire: Etablissement Français du Sang, 93218 La Plaine Saint Denis Cedex (FR)
(72) Inventeur: FOURNIER-WIRTH, Chantal, F-34830 Clapiers (FR); COSTE, Joliette, F-34000 Montpellier (FR)
(74) Mandataire: Vuillermoz, Bruno
(86) Numéro de dépôt international: PCT/FR2007/051023
(87) Numéro de publication internationale: WO 2007/110549

(56) Documents cités:
- WO-A-2005/049637
- FAURSCHOU ET AL.: "Defensin-rich granules of human neutrophils: charcterization of secretory properties" BIOCHIMICA ET BIOPHYSICA ACTA, vol. 1591, 2002, pages 29-35, XP002409956
- PROHAZKA ET. AL.: "Defensins purified from human granulocytes" MOLECULAR IMMUNOLOGY, vol. 34, no. 11, 1997, pages 809-816, XP005609672
- WONG J H ET AL: "Sesquin, a potent defensin-like antimicrobial peptide from ground beans with inhibitory activities toward tumor cells and HIV-1 reverse transcriptase" PEPTIDES, ELSEVIER, AMSTERDAM, US, vol. 26, no. 7, juillet 2005 (2005-07), pages 1120-1126, XP004931282 ISSN: 0196-9781

## Description

### DOMAINE TECHNIQUE

L'invention concerne la purification des α-défensines, peptides synthétisés par les polynucléaires neutrophiles humains.

Cette purification est réalisée à partir de filtres de déleucocytation, sur lesquels sont retenus les leucocytes lors de la préparation des différents produits sanguins labiles.

Grâce à un protocole d'extraction et de purification adapté, il est possible d'obtenir des quantités importantes de peptides actifs, ayant de multiples applications en raison de leurs propriétés bactéricides, virucides, cytotoxiques, immunomodulatrices et chimiotactiques.

Cette source d'α-défensines est particulièrement adaptée en raison de la simplicité et du bon rendement du protocole mis en oeuvre. En outre, c'est une source économique, dans la mesure où les filtres de déleucocytation sont des produits de déchet, voués à la destruction.

### ETAT DE LA TECHNIQUE

Les mammifères sont constamment exposés à un large éventail de microorganismes. Cependant, ils deviennent rarement infectés grâce au rôle de barrière protectrice, joué par la peau et les épithéliums, et à la présence de substances antimicrobiennes (1). Si la barrière est franchie, les pathogènes invasifs sont contrôlés et éliminés ultérieurement par le système immunitaire de l'hôte. Les mammifères ont développés deux types d'immunité : "innée", non clonale et non spécifique, et "adaptative", inductible et spécifique d'antigène.

L'immunité innée (constitutive) représente la première ligne de défense de l'hôte qui est rapidement mobilisée en cas de détection d'une invasion microbienne. Elle repose sur le recrutement et/ou l'activation de leucocytes (granulocytes, monocytes/macrophages...), capables de combattre les pathogènes et sur la libération et/ou l'activation d'une variété de médiateurs humoraux extracellulaires (facteurs du complément, cytokines, substances antimicrobiennes ...).

L'immunité adaptative est induite lorsque les lymphocytes sont activés, en réponse à l'antigène présenté par les cellules présentatrices d'antigène (CPA), en particulier par les cellules dendritiques (CD). Les récepteurs présents sur les lymphocytes T reconnaissent les épitopes antigéniques liés au complexe majeur d'histocompatibilité (CMH) présents à la surface des CPA. Les lymphocytes T CD8+, activés après la reconnaissance des épitopes antigéniques liés au CMH de classe I (sur la plupart des cellules à noyau de l'organisme), se différencient en lymphocytes cytotoxiques qui tuent directement les cellules infectées par les pathogènes. Les lymphocytes T CD4+, activés après la reconnaissance des épitopes antigéniques liés au CMH de classe II (sur macrophages, cellules dendritiques, lymphocyte B...), évoluent en cellules T helper qui, en produisant diverses cytokines, déclenchent l'activation des lymphocytes B (maturation en plasmocytes et stimulation de la sécrétion d'anticorps) et aident les phagocytes à éliminer les pathogènes.

Les réponses immunes innées et adaptatives travaillent donc de concert pour éliminer les envahisseurs microbiens.

Les substances antimicrobiennes comprennent les molécules chimiques microbicides (peroxyde d'hydrogène, oxydes nitrique, ..) et une large variété de protéines antimicrobiennes (défensines, histatines, cathélicidines ...) douées de nombreuses activités biologiques qui favorisent la défense de l'hôte.

Parmi elles, les défensines sont des peptides cationiques de 29 à 42 acides aminés contenant 6 résidus cystéine invariables qui forment 3 ponts disulfure intramoléculaires (2, 3). Ces ponts disulfure sont essentiels aux activités antimicrobiennes et cytotoxiques.

Les deux principales familles de défensines chez les vertébrés, les α- et les β-défensines, diffèrent par la longueur des segments peptidiques entre les 6 résidus cystéines et par l'appariement de ces résidus en ponts disulfures.

A l'heure actuelle, 6 α-défensines humaines (29 à 35 acides aminés) ont été identifiées. Les α-défensines 1-4 sont aussi appelées HNP 1-4 (Human Neutophile Peptides) car isolées à l'origine dans les polynucléaires neutrophiles (PN/granulocytes) (4). Les HNP 1-3 ne diffèrent que par la nature de leur premier acide aminé (5). Les deux autres, HD-5 et -6, sont exprimées principalement par les cellules intestinales de Paneth (6).

Ces défensines ont toutes été purifiées, séquencées, testées pour leurs activités antimicrobiennes et analysées par RMN et cristallographie. Leur ADNc a également été cloné et séquencé **(6, 7).**

Les trois α-défensines HNP-1-3 représentent 99% du contenu en défensines des PN et 30 à 50% des protéines des granules azurophiles **(8).** La quantité de défensines mesurée est de 3 à 5 µg/million de neutrophiles **(9).** La concentration en HNP-1-3 dans le plasma de sujets sains est d'environ 40 ng/ml mais peut atteindre 1 à 100 µg/ml en cas d'infections sévères (7).

Toutes les défensines caractérisées jusqu'à présent présentent des effets antimicrobiens directs **(10).** Le spectre antimicrobien est très large et comprend les bactéries Gram (+) (*Staphylococcus aureus,* ...) et Gram (-) (*Escherichia coli,* ...), les mycobactéries, de nombreux champignons et certains virus enveloppés (HIV, VSV, HSV, Influenza A, WNV ...) **(1-4, 6,10).**

Le mécanisme d'action n'est pas complètement élucidé à ce jour (11). L'interaction entre les défensines et les microorganismes entraînerait une déstabilisation et une perturbation des membranes cellulaires et donc, une augmentation de la perméabilité cellulaire et de la fixation des petites molécules. La topologie polaire des défensines, avec des régions hydrophobes séparées des régions chargées, permettrait leur insertion dans les membranes cellulaires microbiennes qui contiennent plus de résidus chargés négativement que les membranes des cellules mammifères. Cette interaction serait à l'origine de la formation de pores multiples dans la membrane cellulaire ciblée (1, 2).

Il a été démontré très récemment, que les α-défensines peuvent inhiber la toxine létale de *Bacillus anthracis* dans des conditions physiologiques et protéger contre les conséquences fatales de l'anthrax.

Par ailleurs, les α-défensines présentent des effets chimiotactiques.

Les phagocytes, incluant les PN et les monocytes/macrophages, représentent les premières cellules effectrices de la défense innée antimicrobienne après l'entrée des pathogènes dans l'hôte **(1).** Ces phagocytes nécessitent d'être recrutés sur les sites de l'invasion pour lutter contre le pathogène. Le recrutement des leucocytes se produit en présence de chémokines et autres facteurs chimiotactiques.

Les α-défensines ont un effet chimiotactique envers les monocytes, les lymphocytes T naïfs et les cellules dendritiques (CD) immatures, même si aucun récepteur spécifique n'a été identifié **(1-3).** Les CD participent à l'immunité innée et peuvent soit agir directement en phagocytant et tuant le pathogène, soit produire après activation de nombreux médiateurs (cytokines, chémokines ...).

Les α-défensines sont également des inducteurs des médiateurs inflammatoires, en induisant la dégranulation des mastocytes et la libération d'histamine. Les produits des granules mastocytaires augmentent l'influx de neutrophiles et les défensines pourraient donc promouvoir indirectement le recrutement de neutrophiles phagocytaires sur les sites inflammatoires. La dégranulation, cette fois des neutrophiles recrutés, entraîne la libération de défensines et donc une boucle de régulation positive (1).

De plus, les HNP 1-3 pourraient augmenter l'expression des cytokines pro-inflammatoires TNFα et IL1 des monocytes et diminuer celle de l'IL10 ce qui, sur le site de l'infection microbienne, permettrait aussi d'amplifier les réponses inflammatoires (1).

Une fois recrutés sur le site de l'infection, les phagocytes deviennent activés et travaillent avec de nombreuses molécules effectrices pour détruire l'envahisseur microbien. Les α-défensines humaines peuvent aussi directement activer les phagocytes pour faciliter la phagocytose. Enfin elles participent à la régulation de l'activation de la voie classique du système du complément **(1).**

En cas d'infections systémiques, les concentrations plasmatiques en α-défensines peuvent atteindre 100 µg/ml, ce qui permettrait d'interférer avec la production de glucocorticoïdes (puissants immunosupprésseurs). Les défensines pourraient donc aussi faciliter l'immunité antimicrobienne en inhibant la production de ces médiateurs immunosuppresseurs.

En matière d'immunité innée, la contribution *in vivo* des défensines consiste donc à :
- inactiver ou tuer directement les micro-organismes ;
- faciliter la phagocytose ;
- promouvoir le recrutement de phagocytes, de mastocytes et de CD ;
- augmenter la production de cytokines pro-inflammatoires ;
- supprimer les médiateurs anti-inflammatoires et immunosuppresseurs ;
- réguler l'activation du complément.

En outre, les défensines sont des activateurs de l'immunité adaptative.

La mise en place de l'immunité adaptative antimicrobienne commence sur les sites de l'invasion lorsque les antigènes microbiens sont pris en charge par les CD immatures (1). Lors du traitement des antigènes, les CD immatures évoluent en réponse aux médiateurs endogènes et aux produits microbiens et deviennent des CD matures capables de migrer vers les organes lymphoïdes et de stimuler les cellules T naïves Ag-spécifiques. Les défensines, libérées en large quantité en réponse aux stimuli microbiens et inflammatoires, contribuent au recrutement des CD immatures sur les sites d'invasion et à leur maturation. Ainsi, elles jouent un rôle critique pour l'induction des réponses immunes adaptées.

Par ailleurs, les α- et β-défensines sont chimiotactiques pour les lymphocytes et participeraient donc au recrutement des cellules T effectrices CD4+ et CD8+ sur les sites de l'infection microbienne. Les HNP 1-3 sont aussi présentes dans le noyau des cellules T sanguines périphériques ce qui suggère qu'elles pourraient profondément réguler les fonctions des lymphocytes T.

Enfin, l'activité immunostimulatrice des α- et β-défensines a été démontré *in vivo (1).* Comment les défensines pourraient promouvoir l'immunité adaptative dans le contexte d'une infection ? Les défensines facilitent la prise en charge et le traitement des Ags par les cellules dendritiques. Elles favorisent la présentation antigénique en induisant la maturation des CD directement ou indirectement (via la production de TNFα et IL1 par les monocytes/macrophages). Enfin, les défensines facilitent le recrutement des cellules mémoires CD4+ et CD8+ dans les tissus infectés et contribuent donc à la phase effectrice des réponses immunes antimicrobiennes.

En raison des multiples propriétés des défensines, leur purification a fait l'objet de nombreux travaux.

Ceux-ci ont débuté en 1985, avec l'extraction des HNP1-3 à partir de neutrophiles humains **(4).** Le protocole a consisté à isoler les granulocytes à partir d'une leucaphérèse, puis, à extraire un sédiment riche en granules qui a été ensuite soumis à des chromatographies (colonne Biogel P-10 / HPLC), afin de purifier les trois α-défensines. L'activité antimicrobienne (vis-à-vis de *S*. *aureus, P. aeruginose, E.coli*), antifongique (vis-à-vis de C. *neofermentants*) et antivirale (vis-à-vis de *HSV*) du produit de purification a été rapportée. Un travail de microscopie électronique a également permis de localiser les HNP 1-3 dans les granules azurophiles. Simultanément, la structure primaire de ces défensines, constituées de 29 (HNP 2) ou 30 (HNP 1 et 3) acides aminés et ne différant entre elles que par la nature du premier acide aminé, a été décrite **(5).**

En 1994, la préparation et l'extraction des granules, à partir d'une poche de leucaphérèse, de neutrophiles obtenus à partir de buffy coat (couche leuco-plaquettaire) ou de sang total ont été rapportées. Les défensines ont ensuite été purifiées par chromatographie à partir des granules **(12).**

D'après *Shiomi et al.,* la concentration des HNP 1-3 dans le plasma humain est égale à 400 ng/ml contre 13 µg/ml dans le sang total **(12).** On estime entre 3 et 5 µg la quantité de HNP 1-3 pour 1.10⁶ neutrophiles **(8, 9).**

Les défensines présentent la particularité de pouvoir être sécrétées, après stimulation des neutrophiles par le PMA (phorbol myristate acétate). Ainsi, il a été montré qu'à fortes concentrations en PMA (1 µg/ml), 8% des défensines sont libérées **(9).** Le profil de libération des défensines, en fonction de la dose de PMA, est corrélé (bien que plus faible) à celui de la β-glucuronidase et de l'élastase, autres marqueurs des granules azurophiles. Une des explications possibles de la mauvaise détection des défensines dans le milieu extracellulaire, après stimulation par le PMA, pourrait être le fait que les défensines ont une forte affinité pour les surfaces cellulaires. Dans le processus de sécrétion, ces peptides pourraient donc être adsorbés ou bien incorporés dans la membrane cellulaire des neutrophiles.

Le traitement des neutrophiles par l'IL8 (Interleukine 8) à 50 ng/ml entraîne *in vitro* leur dégranulation et la libération des défensines, à hauteur de 10% du contenu en défensines des granules de 2.10⁶ neutrophiles. Pour comparaison et d'après les analyses en western blot par imagerie (scan + NIH image), le traitement par fMLP (10⁻⁷ M) libère 17% du contenu, celui par un mélange PMA (2 ng/ml)/ ionomycine (500 nM) 23% (13).

Les propriétés sécrétoires des neutrophiles ont été étudiées de plus près par l'équipe de Borregaard en 2002 (Faurschou *et al.,* **14**)**.** La libération des α-défensines et des marqueurs classiques des différents types de granules a été étudiée, en réponse à différentes stimulations de neutrophiles isolés (30.10⁶) : Ionomycine (1µM), Cyt B (Cytochalasine B) (5µM)/fMLP (Formyl-méthionyl-leucyl-phénylalanine) (1µM), PMA (5µg/ml) ou fMLP (10 nM). Dans toutes les conditions, l'exocytose des α-défensines et de la myélopéroxidase (granules azuophiles) a été observée au même niveau, tandis que celle de la lactoferrine était légèrement supérieure et celle de la gélatinase la plus élevée. Pour les α-défensines, les meilleurs résultats ont été obtenus avec la stimulation par l'ionomycine ou par l'association CytB/fMLP. Dans les deux cas, environ 20% d'exocytose a été observée.

Le document WO 2005/049637 décrit certes une méthode de purification des défensines à partir de globules blancs, basé sur un protocole relativement complexe : relargage des défensines dans une solution acide, extraction de cette solution puis étapes de purification, notamment chromatographiques.

Comme source de globules blancs, il est mentionné des poches, ou éventuellement des filtres. Il s'agit alors de filtres commerciaux classiques utilisés lors d'une étape supplémentaire de purification au cours de laquelle les globules blancs sont discriminés sur la base de leur taille.

Il existe actuellement un besoin évident de nouveaux protocoles de purification des défensines, molécules d'intérêt thérapeutique majeur notamment en tant qu'antibiotiques à large spectre, dont la synthèse chimique des formes actives est réputée comme particulièrement délicate.

Le problème technique que se propose de résoudre la présente invention est donc de fournir une source et un protocole permettant l'extraction en grandes quantités des défensines, de manière aisée, avec des bons rendements et un coût modéré.

### DESCRIPTION DETAILLEE DE L'INVENTION

Ainsi et selon un premier aspect, l'invention concerne l'utilisation de filtres de déleucocytation pour la purification des défensines.

Les filtres de déleucocytation sont utilisés pour filtrer le sang au cours de la préparation des produits sanguins labiles (PSL), tels que CGR (Concentré de Globules Rouges), CP (Concentré de Plaquettes), PFC (Plasma Frais Congelé) ou MCP (Mélange de Concentrés Plaquettaires). Leur fonction est de débarrasser ces produits sanguins des leucocytes, dont les polynucléaires neutrophiles (PN) représentent la population majoritaire, retenus sur lesdits filtres.

Des filtres de déleucocytation sont disponibles sur le marché. Il peut par exemple s'agir des "filtres pour la déleucocytation de mélanges de concentrés plaquettaires standards à partir de Buffy Coat", commercialisés par la société Pall. Il s'agit de filtres multicouches en polyester assurant la rétention par affinité des leucocytes, le mécanisme d'action précis (par ligand ou par effet de charges) étant gardé secret par les fabricants.

Dans les protocoles de préparation des produits sanguins, les filtres de déleucocyation sont voués à la destruction. Ils constituent donc une source en quantité importante de PN, et donc en défensines, à la fois enrichie et très économique. La présente invention repose donc sur le concept de recyclage biologique. Les défensines pourraient ainsi représenter une nouvelle molécule issue du fractionnement de produits sanguins.

En outre, ces filtres de déleucocytation faisant partie d'une chaîne très contrôlée d'obtention des "médicaments dérivés du sang", ils bénéficient de l'acquis transfusionnel, en particulier des bonnes pratiques de préparation, de l'exigence de traçabilité des produits et des contrôles de l'hémovigilance. Ces filtres de déleucocytation constituent donc une source sanitairement sûre de défensines.

Le document WO 93/16201 proposait d'utiliser les cellules issues d'un échantillon sanguin, retenues sur un filtre de déleucocytation, pour analyser la contamination virale. A cet effet, les cellules du filtre sont lysés pour libérer le génome viral ou les antigènes viraux, et les tests adaptés (PCR ou anticorps) sont menés sur ce matériel.

En outre, le document WO 02/14560 a préconisé l'utilisation de filtres de déleucocytation pour analyser l'ADN génomique des cellules retenues. Selon ce protocole, les cellules sont lysées *in situ.* L'ADN reste accroché au filtre pendant le lavage des débris cellulaires puis est élué sous forme purifié.

Toutefois, aucun document ne décrit l'utilisation de ces filtres pour la préparation de quantités significatives de matériel peptidique actif.

Préférentiellement, les défensines sont d'origine humaine, c'est-à-dire que les filtres de déleucocytation sont ceux récupérés après traitement de sang humain.

Comme mentionné précédemment, les cellules PN ainsi récupérées contiennent uniquement les α-défensines, plus précisément les α-défensines 1 à 4 (HNP 1-4), dont 99% sont constituées par les α-défensines 1 à 3 (HNP 1-3). En outre, en raison de la similarité de séquence et de structure existant entre ces 3 défensines, les protocoles classiques de purification, basés par exemple sur le poids moléculaires ou le pouvoir antigénique, entraînent la copurification des ces trois peptides HNP 1-3.

Le terme "purification", utilisé dans le cadre de la présente invention, a un sens similaire aux termes "extraction" ou "isolement". Il s'agit d'enrichir le matériel biologique en défensines actives, dont la concentration se trouve ainsi augmentée.

A partir de la matière première que constituent les filtres de déleucocytation, riches en PN, une ou plusieurs étapes de purification successives peuvent donc être mises en oeuvre.

Selon un second aspect, l'invention concerne donc un procédé de purification des défensines. Dans une première étape, celui-ci se caractérise par le passage d'un extrait sanguin sur un filtre de déleucocytation et la récupération dudit filtre.

L'extrait sanguin est avantageusement d'origine humaine.

L'extrait sanguin peut être constitué par du sang à l'état brut. De manière préférentielle, il s'agit d'une couche leuco-plaquettaire, également appelée "buffy coat".

Dans une étape ultérieure, le filtre est lavé au moins une fois, préférentiellement 5 à 6 fois, à l'aide d'un tampon salin. Typiquement, le tampon est du PBS (Phosphate Buffer Saline). Avantageusement, ce traitement du filtre de déleucocytation se déroule en milieu stérile, par exemple sous hotte à flux laminaire. Cette étape a pour but de décrocher les PN porteurs dans leurs granules des défensines.

Le volume de tampon de lavage est récolté et est appelé "lysat". Ce lysat contient les cellules ou fragments cellulaires qui se sont détachés du filtre sous l'action du tampon salin.

L'étape suivante consiste à faire sécréter les défensines par les cellules.

La stimulation de la sécrétion des défensines est connue de l'homme du métier. Avantageusement, elle est réalisée en présence de Cyt B (Cytochalasin B) et fMLP (N-formylméthionyl-leucyl-phénylalanine), ajoutés à une concentration micromolaire, préférentiellement comprise entre 1 et 10 µM.

Cette exocytose peut être réalisée sur le lysat entier mais est avantageusement mise en oeuvre sur le culot cellulaire, obtenu après centrifugation du lysat. Cette centrifugation est typiquement réalisée pendant quelques minutes à environ 2500 tr/min.

Afin d'éviter la dégradation des défensines qui ne seront plus protégées par les granules après sécrétion, des inhibiteurs de protéases sont avantageusement ajoutés au mélange réactionnel.

Après arrêt de la réaction et centrifugation (200 g pendant quelques minutes), les défensines sécrétées sont récupérées dans le surnageant:

Afin d'enrichir la fraction ainsi obtenue en α-défensines 1 à 3 (HNP1-3), il est possible de faire passer le surnageant sur une colonne d'immunoprécipitation contenant un anticorps spécifique des HNP1-3. Dans un premier temps, ces peptides sont capturés sur cette colonne en raison de l'affinité antigène-anticorps puis sont élués sous forme purifiée.

Bien sûr, d'éventuelles étapes ultérieures de purification telles que des chromatographies (gel filtration, HPLC...) peuvent être réalisée en fonction du degré de pureté recherché.

L'originalité de ce protocole repose donc sur un traitement chimique direct à partir des filtres de déleucocytation et évite les étapes lourdes de purification de cellules ou de ses organites. En effet, l'exocytose est réalisée directement sur les cellules du filtre de déleucocytation, sans que les granulocytes et les granules azurophiles ne soient isolés. Ainsi, le procédé d'extraction mis en place est très simple ce qui constitue un point fort en terme d'automatisation.

Par ailleurs, le rendement attendu de ce procédé est excellent : en effet, il apparaît qu'à partir d'un filtre de déleucocytation, qui comme déjà dit émane de la filtration d'un pool de concentrés plaquettaires correspondant à 5 ou 6 "buffy coat", il est possible d'obtenir au moins 0,5 µg, avantageusement entre 1 µg et 2 µg, et même jusqu'à 5 µg de défensines HNP1-3 purifiées.

### EXEMPLES DE REALISATION

Les exemples de réalisation décrits ci-dessous ont pour but d'illustrer davantage la présente invention, selon un de ses modes de réalisation privilégiés. Toutefois, ces exemples de réalisation ne sont en aucun cas limitatifs.

### LEGENDE DES FIGURES

- Figure 1 :: Agencement d'un filtre de déleucocytation.
- Figure 2 :: Analyse par Western Blot des défensines HNP 1-3 purifiées à partir de filtres de déleucocytation.
- Figure 3 :: Analyse par SELDI-TOF-MS des défensines HNP1-3 purifiées à partir de filtres de déleucocytation.
- Figure 4 :: Analyse par SELDI-TOF-MS (zoom) des défensines HNP1-3 purifiées à partir de filtres de déleucocytation.

### 1/ Préparation des MCP :

Les mélanges de concentrés plaquettaires (MCP) sont préparés classiquement selon la méthode des "buffy-coat". Un prélèvement de sang total d'un donneur de sang est effectué selon les bonnes pratiques transfusionnelles, puis stocké pendant 15 à 20 heures à température ambiante avant la préparation des différents produits sanguins. Le sang total est alors centrifugé à 4000 trs/min pendant 20 min à 20°C et séparé en globules rouges, plasma et couche leuco-plaquettaire dite "buffy coat" (contenant les plaquettes, les leucocytes et 45 g de plasma). Après séparation, 5 à 6 "buffy coats" compatibles sont rassemblés et centrifugés à faible vitesse (1700 trs/min pendant 11 min à 20°C). Le surnageant contenant les plaquettes est alors filtré, afin de retenir les leucocytes, et transféré dans une poche de stockage définitive qui sera ensuite distribuée comme "MCP" déleucocyté.

L'ensemble des étapes est réalisé en circuit fermé à l'aide d'un kit de préparation à usage unique comprenant plusieurs poches. Le rassemblement des différents "buffy coats" mélangés est réalisé par connections stériles entre les différentes poches unitaires. Le filtre de déleucocytation utilisé dans l'étape terminale avant transfert dans la poche de stockage constitue la source initiale de défensines. En pratique, il s'agit d'un filtre de déleucocytation commercialisé par la société PALL sous la référence ***Pall A TSBC2PSF "AutoStop BC Pall".***

### 2/ Traitement du filtre de déleucocytation :

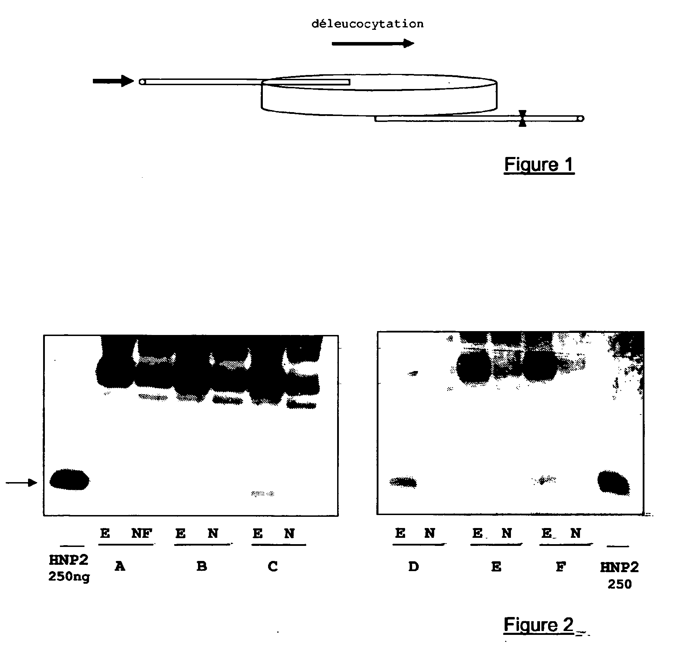

Un filtre de déleucocytation, intégré dans un circuit fermé de traitement des produits sanguins, se présente comme illustré à la figure 1.

Après avoir effectué la filtration terminale du MCP, le filtre est isolé du kit par 2 soudures stériles effectuées sur les tubulures en amont et en aval de celui-ci. Le filtre conçu en plastique rigide ne peut être ouvert.

Le filtre est alors traité directement dans des conditions stériles, sous hotte à flux laminaire : la solution A *(Phosphate Buffer Saline (PBS 1X), 3 ml)* est injectée à l'aide d'une seringue introduite dans la tubulure amont. Le filtre est rincé par aspiration, à raison de 6 aller-retours effectués avec la seringue. Le lysat est collecté dans un tube.

### 3/ Purification des défensines :

Trois étapes courtes sont effectuées successivement :

### • centrifugation

Le lysat (2 ml), obtenu après lavage du filtre; est centrifugé à 2500 trs/min pendant 10 minutes à 10°C.

### • exocytose

Le surnageant obtenu est éliminé. La stimulation est réalisée sur le culot cellulaire, après ajout d'inhibiteurs de protéases (*Complete Tablets, Roche ;* 112 µl) et de 160 µl de tampon Krebs-Ringer *(130 mM NaCl, 5 mM KCl, 1,27 mM MgSO₄, 0,95 mM CaCl₂, 5 mM glucose, 10 mM NaH₂PO₄*/*Na₂HPO₄ pH 7,4*), par un traitement pendant 5 min à 37°C à la Cytochalasine B (*Sigma, 2.10⁻⁵* M ; 104 µl soit 5,53 µM) suivi d'une incubation pendant 15 min à 37°C en présence de N-formylméthionyl-leucyl-phénylalanine (fMLP, *Sigma, 11,4.10⁻⁶* M ; 43 µl soit 1.17 µM). La réaction est arrêtée par une dilution avec du tampon KRP froid (531 µl). Après centrifugation (200 g pendant 6 minutes), le surnageant obtenu est réparti en 4 tubes de 240 microlitres et peut être conservé à -80°C. Ce protocole est adapté de celui décrit par Faurshou *et al. (14).*

### • immunoprécipatation

Chaque aliquot obtenu après exocytose est traité par immunoprécipitation afin de purifier les α-défensines HNP 1-3.

Les immunoprécipations sont réalisées à l'aide du système µ*Macs Protein G Microbeads* (*Miltenyi Biotec*). L'anticorps monoclonal *Purified Mouse anti-human α-defensin 1-3 Monoclonal Antibody (BD Pharmingen)* est utilisé pour la capture.

En pratique, 240 µl de surnageant (produit d'exocytose) sont mélangés à 5 µl d'anticorps anti HNP 1-3 (0,2 mg/ml), 50 (µl de billes magnétiques et 700 µl de tampon de lyse (*150 mM NaCl, 1% Triton X100, 50 mM Tris HCl pH 8).* Le tout est incubé dans la glace pendant 75 min. Ensuite, le mélange est déposé sur colonne µMACS préhydratée avec 200 µl de tampon de lyse. La colonne est lavée 4 fois avec 200 µl de tampon de lavage "High Salt Buffer" *(500 mM NaCl, 1% NP-40, 50 mM Tris HCl pH 8),* puis 1 fois avec 100 µl de tampon de lavage "Low Salt Buffer" *(20 mM Tris HCl pH 7,5).* Ensuite, 20 µl de tampon d'élution *(50 mM Tris HCl pH 6,8, 1% SDS)* sont préchauffés à 95°C, appliqués et laissés 10 min en contact. L'élution est réalisée à l'aide de 50 µl de tampon d'élution préchauffés à 95°C.

L'éluat obtenu de chaque colonne d'immunoprécipitation (45 microlitres au final en moyenne) est conservé à -20 °C avant analyse. 30 µl servent à l'analyse par Western blot en présence de 10 µl de tampon de dépôt (*NuPage Invitrogen LDS 4X).*

### 4/ Analyse des éluats HNP1-3 obtenus après immunoprécipitation :

### 4.1. Référence :

Une défensine commerciale HNP2 humaine purifiée (≥ *95% HPLC*/*3370,95 Da, Sigma)* a été utilisée comme référence dans les essais.

Un contrôle de séquence (séquençage NH2 terminal chimique) a été réalisé par la plateforme d'Analyse et de Microséquençage des Protéines de l'Institut Pasteur à Paris.

### 4.2. Détection par Western Blot

Les protéines éluées sont séparées en condition non réductrice par électrophorèse monodimensionnelle en gel d'acrylamide *(NuPAGE Bis-tris 12% acrylamide, Invitrogen)* et transférées sur membrane de nitrocellulose *(Hybond ECL, Amersham).* L'anticorps monoclonal *"Purified Mouse anti-human α-defensin 1-3 Monoclonal Antibody"* (0,2 µg/µl, *BD Pharmingen)* est utilisé pour la reconnaissance des défensines HNP1-3. La révélation est réalisée à l'aide d'un kit d'immunodétection chemilumiscent *(WesternBreeze, Invitrogen)* selon les recommandations du fournisseur.

La structure primaire des α-défensines a été décrite en 1985 par l'équipe de R. Lehrer (5). Ces protéines sont constituées de 29 (HNP2) ou 30 (HNP 1 et 3) acides aminés et ne diffèrent entre elles que par la nature du premier acide aminé. Ainsi, l'anticorps monoclonal commercial choisi est utilisé pour l'immunoprécipitation et la détection de ces trois défensines, mais ne permet pas de les discriminer entre elles.

6 filtres (A à F) de déleucocytation de mélanges de concentrés plaquettaires (MCP) préparés selon la méthode des « Buffy Coat » en vue de la purification des α-défensines ont été traités (Figure 2). Après immunoprécipitation à l'aide de l'anticorps monoclonal anti-HNP1-3, les éluats obtenus (E) ont chacun été analysés par Western Blot. Les fractions non fixées (NF) sont utilisées comme contrôle négatif. La défensine de synthèse HNP2 *(Sigma)* est utilisé comme contrôle positif du Western Blot.

A l'aide de l'anticorps monoclonal anti-HNP 1-3, la présence de protéines aux masses attendues est détectée dans les éluats obtenus après immunoprécipitation (E). Ces protéines ne sont pas détectées dans les premières fractions obtenues lors du passage du lysat sur la colonne (fractions non fixées, NF).

Une estimation quantitative des signaux a été réalisée par logiciel d'analyse d'image *(SigmaGel Software)* sur l'ensemble des profils obtenus par Western Blot. Ainsi, il a pu être estimé que le traitement rapide par exocytose des cellules obtenues après centrifugation de 2 ml de lysat (issu du lavage d'un filtre) permet de libérer entre 0,5 µg et 1,5 µg de défensines HNP1-3 purifiées.

### 4.3. Détection par spectrométrie SELDI-TOF-MS (Ciphergen Biosystem)

La Technologie SELDI-TOF-MS ProteinChip CIPHERGEN *(Palo Alto, CA)* (www.ciphergen.com) combine la capture de protéines sur des surfaces chimiques actives et l'analyse par spectrométrie de masse en temps de vol (TOF-MS). Elle permet d'analyser directement des protéines à partir de liquides biologiques complexes et nécessite de très petites quantités pour l'analyse (micro-échantillonage). Cette technologie permet la séparation, la détection et l'analyse de protéines au niveau femtomolaire. Elle améliore la découverte de protéines inférieures à 30kDa et est insensible au point isoélectrique de celles-ci.

L'originalité de la plate-forme ProteinChip réside, d'une part, dans l'existence de différentes barrettes permettant la séparation et la capture des protéines en fonction de propriétés chimiques ou biochimiques des protéines (hydrophobes, anioniques, cationiques, ...) et d'autre part dans le logiciel d'analyse de données permettant la comparaison des profils protéiques.

Les applications de cette technologie sont diverses : étude de l'expression différentielle de protéines, reconnaissance moléculaire, purification et caractérisation de protéines, validation et identification de marqueurs.

Cette technologie a été utilisée par Zhang *et al.* **(15)** pour l'étude des défensines humaines HNP1-3 en tant que molécules antivirales anti-HIV et plus récemment par Albrethsen et *al* **(16)** dans une approche de protéomique visant à étudier l'expression des HNP1-3 dans le cancer du colon.

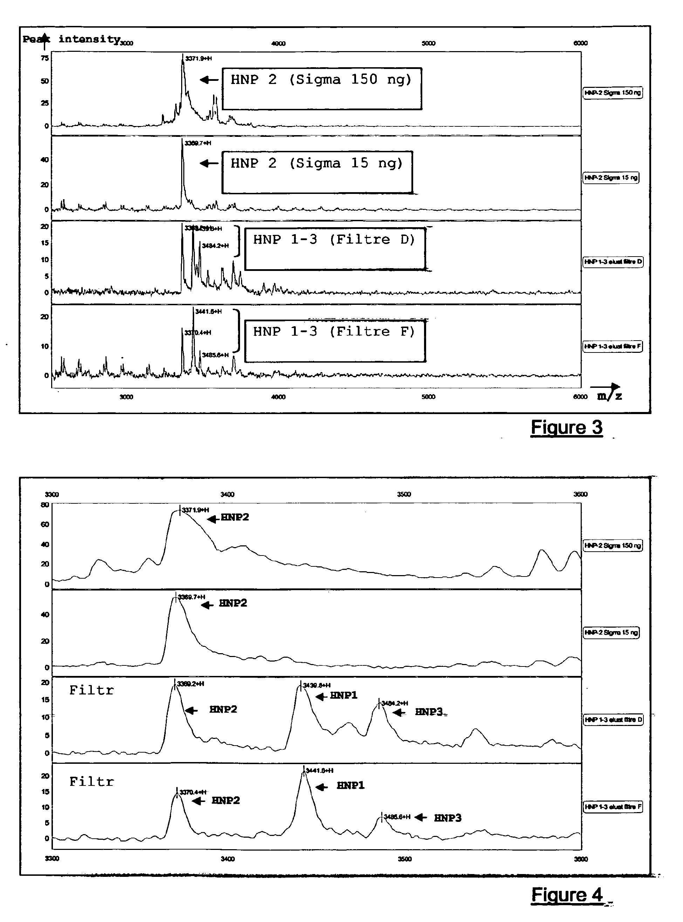

L'éluat d'immunoprécipitation (3 µl) est analysé directement sur une barrette de type NP20 *(NP20 Proteinchip array, normal phase, Ciphergen).* Après séchage (20 min), les protéines non fixées sont éliminées par deux lavages successifs et un temps de séchage de 5 à 10 min est observé avant l'ajout de la matrice (SPA, 50% acétonitrile, 0,5% TFA) en vue de la désorption des protéines. La barrette est alors placée dans la plateforme d'analyse (*Ciphergen ProteinChip Reader PBSII).* Les protéines complexées à la matrice sont désorbées par le laser, leur temps de vol est proportionnel à leur ratio masse/charge (m/z). Les pics détectés sont représentés sur la Figure 3, et de manière zoomée à la Figure 4.

L'ensemble des pics a été analysé après calibration à l'aide du mix de calibration *"All in one"* peptide molecular weight standards (*Ciphergen Biosystems)* dans les conditions préconisées par le fournisseur.

Les critères de lecture appliqués ont été les suivants : laser intensity 180 / detector sensitivity 9 / High Mass 10000, optimized from 1000 to 7500 / Focus mass : 3400.

Il a ainsi été confirmé la présence de protéines avec des ratios masse/charge de 3370, 3440 et 3485 dans les éluats d'immunoprécipitation. Ces peptides correspondent respectivement aux défensines HNP2, HNP1 et HNP3.

Ces résultats sont en accord avec les spectres obtenus par SELDI- TOF- MS dans l'article de Zhang *et al.* (3372/ 3442/3486 ; **15**) et dans celui d'Albrethsen *et al.* (3372/ 3443/3486 ; **16**).

Les résultats obtenus permettent donc de valider la possibilité de purifier des défensines alpha HNP 1-3 à partir de filtres de déleucocytation de mélanges de concentrés plaquettaires.

### BIBLIOGRAPHIE

1. Yang D, Biragyn A, Hoover DM, Lubkowski J, Oppenheim JJ. Multiple rôles of antimicrobial defensins, cathelicidins, and eosinophil-derived neurotoxin in host defense. Annu Rev Immunol 2004;22:181-215.
2. Ganz T. Defensins: antimicrobial peptides of innate immunity. Nat Rev Immunol 2003;3:710-720.
3. Yang D, Biragyn A, Kwak LW, Oppenheim JJ. Mammalian defensins in immunity: more than just microbicidal. Trends Immunol 2002;23:291-296.
4. Ganz T, Selsted ME, Szklarek D, Harwig SS, Daher K, Bainton DF, Lehrer RI. Defensins. Natural peptide antibiotics of human neutrophils. J Clin Invest 1985;76:1427-1435.
5. Selsted ME, Harwig SS, Ganz T, Schilling JW, Lehrer RI. Primary structures of three human neutrophil defensins. J Clin Invest 1985;76:1436-1439.
6. Lehrer RI, Lichtenstein AK, Ganz T. Defensins: antimicrobial and cytotoxic peptides of mammalian cells. Annu Rev Immunol 1993;11:105-128.
7. Ganz T, Lehrer RI. Defensins. Pharmacol Ther 1995;66:191-205:
8. Shiomi K, Nakazato M, Ihi T, Kangawa K, Matsuo H, Matsukura S. Establishment of radioimmunoassay for human neutrophil peptides and their increases in plasma and neutrophil in infection. Biochem Biophys Res Commun 1993;195:1336-1344.
9. Ganz T. Extracellular release of antimicrobial defensins by human polymorphonuclear leukocytes. Infect Immun 1987;55:568-571.
10. Ganz T, Lehrer RI. Antimicrobial peptides of vertebrates. Curr Opin Immunol 1998;10:41-44.
11. Selsted M, Ouellette AJ. Mammalian defensins in the antimicrobial immune response. Nature Immunol. 2005;6:551-557.
12. Harwig SS, Ganz T, Lehrer RI. Neutrophil defensins: purification, characterization, and antimicrobial testing. Methods Enzymol 1994;236:160-172.
13. Chertov O, Michiel DF, Xu L, Wang JM, Tani K, Murphy WJ, Longo DL, et al. Identification of defensin-1, defensin-2, and CAP37/azurocidin as T-cell chemoattractant proteins released from interleukin-8-stimulated neutrophils. J Biol Chem 1996;271:2935-2940.
14. Faurschou M, Sorensen OE, Johnsen AH, Askaa J, Borregaard N. Defensin-rich granules of human neutrophils: characterization of secretory properties. Biochim Biophys Acta 2002;1591:29-35.
15. Zhang L, Yu W, He T, et al. Contribution of human alpha-defensin 1, 2, and 3 to the anti-HIV-1 activity of CD8 antiviral factor. Science. 2002;298:995-1000.
16. Albrethsen J, Bogebo R, Gammeltoft S, Olsen J, Winther B, Raskov H. Upregulated expression of human neutrophil peptides 1, 2 and 3 (HNP 1-3) in colon cancer serum and tumours: a biomarker study. BMC Cancer. 2005;5:8.

## Revendications

1. Procédé de purification des défensines, ***caractérisé* en ce que** les défensines sont purifiées à partir d'un filtre de déleucocytation riche en polynucléaires neutrophiles.

2. Procédé de purification des défensines selon la revendication 1, comprenant les étapes suivantes :
- le passage d'un extrait sanguin sur un filtre de déleucocytation ;
- le décrochage des cellules retenues sur le filtre de déleucocytation.
- l'induction de l'exocytose des défensines par ces cellules.

3. Procédé de purification des défensines selon la revendication 2, ***caractérisé* en ce que** l'extrait sanguin est une couche leuco-plaquettaire.

4. Procédé de purification des défensines selon la revendication 2 ou 3, ***caractérisé* en ce que** le décrochage des cellules retenues sur le filtre de déleucocytation est réalisé par lavage à l'aide d'un tampon salin.

5. Procédé de purification des défensines selon l'une des revendications 2 à 4, ***caractérisé* en ce que** l'induction de l'exocytose est réalisée sur le culot cellulaire, obtenu après centrifugation du produit de lavage du filtre de déleucocytation.

6. Procédé de purification des défensines selon l'une des revendications 2 à 5, ***caractérisé* en ce que** l'induction de l'exocytose est réalisée à l'aide de Cyt B et de fMLP.

7. Procédé de purification des défensines selon l'une des revendications 2 à 6, ***caractérisé* en ce qu'**après l'exocytose, une centrifugation est réalisée et le surnageant est récolté.

8. Procédé de purification des défensines selon la revendication 7, ***caractérisé* en ce qu'**après l'exocytose, une immunoprécipitation est réalisée sur le surnageant.

9. Procédé de purification des défensines selon l'une des revendications 2 à 8, ***caractérisé* en ce que** l'extrait sanguin est d'origine humaine et que les défensines purifiées sont les HNP1-3.

## Patentansprüche

1. Aufreinigungsverfahren von Defensinen, ***dadurch gekennzeichnet,* dass** die Defensinen ausgehend von einem Deleukozytationsfilter aufgereinigt werden, der reich an neutrophilen polynuklearen Zellen ist.

2. Aufreinigungsverfahren von Defensinen gemäß Anspruch 1, umfassend die folgenden Stufen:
- Den Durchgang eines Blutextrakts auf einem Deleukozytationsfilter;
- Das Ablösen der auf dem Deleukozytationsfilter zurückgehaltenen Zellen,
- Die Induktion der Exozytose der Defensinen durch diese Zellen.

3. Aufreinigungsverfahren von Defensinen gemäß Anspruch 2, ***dadurch gekennzeichnet,* dass** der Blutextrakt eine Leukozytenschicht ist.

4. Aufreinigungsverfahren von Defensinen gemäß Anspruch 2 oder 3, ***dadurch gekennzeichnet,* dass** das Ablösen der auf dem Deleukozytationsfilter zurückgehaltenen Zellen per Waschen mithilfe eines salzigen Puffers realisiert wird.

5. Aufreinigungsverfahren von Defensinen gemäß Anspruch 2 bis 4, ***dadurch gekennzeichnet,* dass** die Induktion der Exozytose auf dem Zellkonzentrat realisiert wird, das nach dem Zentrifugieren des Waschungsprodukts des Deleukozytationsfilters erhalten wird.

6. Aufreinigungsverfahren von Defensinen gemäß Anspruch 2 bis 5, ***dadurch gekennzeichnet,* dass** die Induktion der Exozytose mithilfe von Cyt B und fMLP realisiert wird.

7. Aufreinigungsverfahren von Defensinen gemäß Anspruch 2 bis 6, ***dadurch gekennzeichnet,* dass** nach der Exozytose eine Zentrifugation realisiert wird und der Überstand abgeschöpft wird.

8. Aufreinigungsverfahren der Defensinen gemäß Anspruch 7, ***dadurch gekennzeichnet,* dass** nach der Exozytose auf dem Überstand eine Immunpräzipitation realisiert wird.

9. Aufreinigungsverfahren von Defensinen gemäß Anspruch 2 bis 8, ***dadurch gekennzeichnet,* dass** der Blutextrakt menschlichen Ursprungs ist und dass die aufgereinigten Defensinen die HNNP1-3 sind.

## Claims

1. Defensin purification method ***characterised* in that** the defensins are purified from a deleukocytation filter.

2. Defensin purification method as claimed in claim 1, including the following steps:
- running a blood extract through a deleukocytation filter;
- releasing the cells trapped on the deleukocytation filter;
- inducing defensin exocytosis by these cells.

3. Defensin purification method as claimed in claim 2, ***characterised* in that** the blood extract is a leukocyte-platelet layer.

4. Defensin purification method as claimed in claim 2 or 3, ***characterised* in that** the release of the cells trapped on the deleukocytation filter is achieved by washing with a saline buffer.

5. Defensin purification method as claimed in any of claims 2 to 4, ***characterised* in that** exocytosis is induced on the cell pellet obtained after centrifugation of the washing product from the deleukocytation filter.

6. Defensin purification method as claimed in any of claims 2 to 5, ***characterised* in that** exocytosis is induced using Cyt B and fMLP.

7. Defensin purification method as claimed in any of claims 2 to 6, ***characterised* in that**, after exocytosis, centrifugation is performed and the supernatant is collected.

8. Defensin purification method as claimed in claim 7 ***characterised* in that**, after exocytosis, immunoprecipitation is performed on the supernatant.

9. Defensin purification method as claimed in any of claims 2 to 8, ***characterised* in that** the blood extract is of human origin and the purified defensins are HNP 1-3.
